# DEMANDE DE BREVET EUROPEEN

(11) **EP 4 140 978 A1**
(43) Date de publication de la demande: **01.03.2023**
(21) Numéro de dépôt: 21193244.7
(22) Date de dépôt: 26.08.2021
(51) Int. Cl.: C07C 51/377, C07C 57/04, B01J 21/00

(54) **PROCÉDÉ DE PRÉPARATION D'ACIDE ACRYLIQUE OU D'ESTER D'ACIDE ACRYLIQUE PAR DÉSHYDRATATION CATALYTIQUE À PARTIR D'UN FLUX DE RECYCLAGE D'UN PROCÉDÉ DE PRODUCTION D'ACIDE POLYLACTIQUE**

(71) Demandeur: Futerro S.A., 7760 Escanaffles (BE)
(72) Inventeur: COSZACH, Philippe, 7760 Escanaffles (BE); POMMIE, Aurélie, 7760 Escanaffles (BE)
(74) Mandataire: AWA Benelux

(57) **Abrégé**

On décrit un procédé de préparation d'acide acrylique ou d'ester d'acide acrylique par déshydratation catalytique d'acide lactique ou d'ester d'acide lactique à une température comprise entre 270 et 400°C en présence d'un catalyseur usuel caractérisé en ce que la charge d'acide lactique ou d'ester d'acide lactique à déshydrater est constituée par un mélange d'acide lactique obtenu par fermentation directe et de 5 à 100% en poids d'acide lactique ou d'ester d'acide lactique provenant des étapes de recyclage d'un procédé de préparation de PLA.

## Description

### Domaine de l'invention

La présente invention se rapporte à un procédé de déshydratation catalytique d'acide lactique ou d'ester d'acide lactique, afin de préparer de l'acide acrylique ou un ester d'acide acrylique.

En particulier le procédé consiste à utiliser une charge particulière d'acide lactique ou d'ester d'acide lactique, provenant des étapes de recyclage d'un procédé de préparation de PLA.

### Etat de l'art

L'acide acrylique est une molécule plateforme utilisé principalement dans l'industrie des polymères avec un marché qui représente 4 Mt/an. Environ 72% de l'acide acrylique produit est utilisé pour la synthèse d'esters acryliques, 24 % pour la fabrication de polymères acryliques et enfin 4% pour des applications diverses telles que des copolymères. Les produits ainsi formés ont des applications industrielles très importantes, en particulier lorsqu'on les utilise sous forme de polymère comme le polyacrylate. On peut notamment citer des applications dans les domaines de la colle, du textile, du revêtement, des résines, dans les peintures, les adhésifs, les liants, les détergents, les floculants, les dispersants et autres analogues.

Le procédé le plus connu de préparation d'acide acrylique est d'effectuer l'oxydation de propène. Or la demande globale en propène est en constante augmentation. Sa principale application étant le polypropylène, un polymère très largement utilisé pour des emballages, plastiques etc. La part du marché de l'acide acrylique dans l'utilisation du propène est très faible. Par conséquent, l'augmentation de la demande en propène qui entraine une augmentation de son prix, rend nécessaire le développement d'une voie de production d'acide acrylique alternative.

De plus, le propène est un dérivé du pétrole et donc non-renouvelable et sa production comme sa conversion contribue à générer du CO2, ce qui n'est pas indiqué par son impact sur l'effet de serre.

Il existe cependant d'autres procédés de préparation d'acide acrylique qui sont plus respectueux de l'environnement comme la déshydratation catalytique d'acide lactique, qui lui est un produit non dérivé du pétrole.

Selon l'état de la technique, il est connu que de l'acide lactique ou un ester d'acide lactique peut être produit par hydrolyse de lactide ou encore d'acide polylactique (PLA) que l'on peut récupérer lors des différentes opérations de recyclage de certains flux apparaissant lors des étapes successives du procédé de préparation de l'acide polylactique.

Ces opérations de recyclage sont effectuées afin de maintenir un bon rendement en acide polylactique.

On peut citer par exemple le brevet WO2015/086613 de la demanderesse, qui décrit un procédé dans lequel plusieurs sous-produits de la production de PLA via le procédé comprenant l'oligomérisation, la cyclisation et la polymérisation par ouverture de cycle, sont réutilisés dans la synthèse de PLA. Les sous-produits recyclés sont notamment l'acide lactique n'ayant pas réagi, les oligomères de faible poids et/ou n'ayant pas réagi et le lactide. Une partie des sous-produits est convertie en acide lactique avant réutilisation, au moyen d'étapes de transestérification, de distillation et d'hydrolyse.

Les brevets US8430948 et US9700840 décrivent également des procédés de recyclage de flux gazeux contenant notamment du lactide permettant de retransformer ce lactide en acide lactique.

La demande de brevet WO2014/180836 décrit un procédé de purification de lactide à utiliser par exemple dans la synthèse d'acide L-polylactique. Un courant de lactide purifié comprenant du L-lactide et du méso-lactide est obtenu à partir d'un lactide brut comprenant du L-lactide, du D-lactide et du méso-lactide. Le procédé de purification de lactide comprend une première étape de distillation, une étape de cristallisation en fusion et une seconde étape de distillation. Le procédé de purification du lactide permet de réduire la quantité de sous-produits indésirables du fait de la récupération d'une grande partie du L-lactide et d'au moins une partie du méso-lactide dans le lactide brut.

L'acide lactique possède deux énantiomères optiquement actifs, le D-acide lactique et le L-acide lactique et donc trois stéréoisomères de lactide peuvent être générés : le L-lactide, le D-lactide et le méso-lactide qui peuvent mener à trois formes stéréochimiques d'acide polylactique : le D-PLA, le L-PLA et un mélange racémique appelé D,L-PLA. Ces trois formes d'acide polylactique ont des propriétés différentes. Il est par exemple connu que la présence d'énantiomère D- dans du L-PLA entraine une diminution de la température de transition vitreuse.

Il est connu que tous les procédés de production de PLA génèrent des sous-produits et induisent des réactions de racémisation. Dans un procédé de production de L-PLA cela a pour résultat d'augmenter la teneur en énantiomère -D- dans le système et inversement, dans un procédé de production de D-PLA, c'est la teneur en énantiomère -L- qui est augmentée.

L'accumulation d'énantiomère -D- ou -L- (selon qu'il s'agit de production de L-PLA ou de D-PLA) dans le système a un impact, non pas sur la pureté chimique du PLA, mais sur sa pureté optique. Par exemple, à certains moments la teneur en énantiomère -D- peut atteindre plusieurs dizaines de pourcent par rapport aux deux énantiomères ce qui a pour effet de réduire considérablement la pureté optique du L-PLA qui serait obtenu avec cet acide lactique. A une telle pureté optique le PLA qui en résulte ne répond plus aux propriétés désirées.

On comprend très bien que les courants résiduels de la production de L-PLA comprennent des quantités substantielles de composés ayant une D-énantiométrie (isométrie optique), tels que l'acide D-lactique, les oligomères d'acide D-lactique, les D-lactides, les méso-lactides, et le D,L-PLA. Et donc le fait de recycler ces courants pour les retransformer en acide lactique va contribuer à accroître la teneur en énantiomère -D-, conduisant dès lors à une perte de rendement ; de plus, si on les laisse s'accumuler, on va vers une diminution drastique de la pureté optique du L-PLA obtenu.

Par réciprocité, il est évident que les courants résiduels de la production de D-PLA comprennent les mêmes composés mais ayant l'isométrie optique -L-.

Cependant, vu la quantité croissante de production de PLA, la quantité d'acide lactique obtenue via les différentes étapes de recyclage des courants résiduels ne fait que croître. Il existe donc un besoin de trouver une voie permettant de limiter la perte en acide lactique de manière drastique et de le recycler sans contrainte tout en permettant un rendement global très élevé du procédé de production de PLA.

### Résumé de l'invention

Un objet de la présente invention est de surmonter un ou plusieurs des inconvénients ci-dessus. L'invention a pour but de proposer un procédé de préparation d'acide acrylique ou d'ester d'acide acrylique, sans inconvénient aucun, par déshydratation catalytique d'acide lactique ou d'ester d'acide lactique, à une température comprise entre 270 et 400°C en présence d'un catalyseur usuel et à raison de 0,1 à 50 moles de vapeur par mole d'acide lactique ou d'ester d'acide lactique, comprenant en tout ou partie de l'acide lactique et/ou des esters d'acide lactique, résultant des différentes étapes de recyclage d'un procédé de préparation de PLA.

La proportion d'acide lactique ou d'ester d'acide lactique provenant du recyclage du procédé de préparation de PLA représente de 5 à 100% en poids et de préférence de 50 à 100% en poids par rapport à la quantité totale d'acide lactique ou d'ester d'acide lactique utilisée pour le procédé de déshydratation en acide acrylique ou ester d'acide acrylique.

### Description détaillée de l'invention

La demanderesse a maintenant trouvé que l'on pouvait préparer de l'acide acrylique ou un ester d'acide acrylique sans inconvénient aucun à partir de flux d'acide lactique ou d'ester d'acide lactique obtenus lors des différentes étapes d'un procédé de production de PLA.

Selon un aspect de l'invention, il est proposé un procédé de valorisation d'un flux d'acide lactique ou d'ester d'acide lactique issu d'un procédé de production de L-PLA contenant de l'acide lactique -D- et/ou un ou plusieurs esters d'acide lactique -D-.

Selon le procédé de la présente invention, on utilise au moins partiellement l'acide lactique et/ou le ou les ester(s) d'acide lactique obtenu(s) par recyclage lors des différentes étapes de préparation de PLA, seul ou en mélange avec de l'acide lactique obtenu par fermentation directe comme base pour la synthèse d'acide acrylique ou d'ester d'acide acrylique. La charge d'acide lactique ou d'ester d'acide lactique utilisée peut contenir de 5% à 100% en poids d'acide lactique ou d'ester d'acide lactique obtenu comme produit résiduel ou sous-produit dans la production de PLA par rapport au poids total de la charge d'acide lactique ou d'ester d'acide lactique, préférentiellement de 50 à 100% en poids d'acide lactique ou d'ester lactique provenant des étapes de recyclage d'un procédé de préparation de PLA par rapport au poids total de la charge d'acide lactique ou d'ester d'acide lactique.

Selon la présente invention, la charge d'acide lactique ou d'ester d'acide lactique provenant des étapes de recyclage du procédé de production de PLA est convertie en acide acrylique ou ester d'acide acrylique par n'importe quel procédé connu de l'homme de l'art.

Selon un mode préféré de l'invention, la charge d'acide lactique provenant des étapes de recyclage du procédé de production de PLA est d'abord convertie en ester d'acide lactique comme par exemple, le lactate d'éthyle par toute technique connue de l'homme de l'art avant d'être convertie en acide acrylique.

Le lactate d'éthyle, en mélange avec de l'eau, est chauffé à une température de 300 à 350°C afin de synthétiser l'acide acrylique, par mise en contact en phase vapeur, à raison de 0,1 à 50 moles, de préférence de 1 à 10 moles de vapeur par mole de lactate d'éthyle, en présence d'un catalyseur.

Il est bien connu que de nombreux catalyseurs existent pour effectuer la déshydratation de l'acide lactique ; on sélectionnera donc ceux qui donnent un bon taux de conversion avec une bonne sélectivité en acide acrylique.

A cet effet, ces catalyseurs sont choisis dans le groupe consistant en les zéolithes de type NaY traitées avec 2% de Ba ou avec NaOH ainsi que des catalyseurs choisis parmi les dérivés phosphatés de l'aluminium ou des zéolithes de type ZSM-5 échangées avec de l'acide phosphorique ou tout autre catalyseur connu pour la déshydratation de l'acide lactique en acide acrylique.

Il est à noter cependant que les zéolithes de type ZSM-5 échangées ou pas permettent un très bon taux de conversion mais avec une sélectivité plutôt tournée vers l'acétaldéhyde que vers l'acide acrylique.

Les catalyseurs utilisés dans le procédé de l'invention sont bien connus pour être utilisés dans une réaction de déshydratation d'acide lactique ou d'ester d'acide lactique et sont choisis parmi ceux décrits dans les brevets US 4786756 ou encore US 9714208.

La présente invention est également illustrée au moyen des exemples suivants qui n'en limitent pas la portée.

### Exemple 1 :

On a préparé selon la méthode décrite dans le brevet US 4786756 un catalyseur de déshydratation d'acide lactique constitué de phosphate d'aluminium sous forme solide.

On a injecté un mélange comprenant de l'acide lactique D-, provenant uniquement du recyclage d'un procédé de production de L-PLA, d'eau et d'azote dans ces proportions molaires (1/18/7) dans un réacteur de déshydratation chauffé à 340°C.

On a obtenu de l'acide acrylique avec un rendement de 43.3%, tandis que la formation d'acétaldéhyde et d'acide propionique étaient plus faibles, respectivement 34.7% et 3.2%.

### Exemple 2

On a utilisé le même catalyseur de déshydratation que dans l'exemple 1.

On a injecté un mélange comprenant 50/50 acide lactique de recyclage/acide lactique obtenu par fermentation directe, d'eau et d'azote dans les mêmes proportions molaires que dans l'exemple 1.

On a obtenu de l'acide acrylique avec un rendement de 42.6%, tandis que la formation d'acétaldéhyde et d'acide propionique étaient plus faibles, respectivement 35.3% et 3.3%.

### Exemple 3

On a injecté un mélange comprenant de l'acide lactique provenant uniquement du recyclage d'un procédé de PLA, que l'on a d'abord transformé en lactate d'éthyle par un procédé connu, ainsi que de l'eau et de l'azote à raison de 30 cm³/min et comportant 6,95% molaire de lactate d'éthyle dans le réacteur de déshydratation chauffé à 300°C, en présence d'un catalyseur de type zéolithe NaY traitée avec 2% de Ba.

La conversion de la réaction a atteint 59% avec une sélectivité en acide acrylique de 47% tandis que l'on a formé également 6,6% d'acétaldéhyde, 1,5% d'acrylate d'éthyle et 0,3% de 2,3-pentanedione.

## Revendications

1. Procédé de préparation d'acide acrylique ou d'ester d'acide acrylique par déshydratation catalytique d'acide lactique ou d'ester d'acide lactique à une température comprise entre 270 et 400°C en présence d'un catalyseur usuel et à raison de 0,1 à 50 moles de vapeur par mole d'acide lactique ou d'ester d'acide lactique, **caractérisé en ce que** la charge d'acide lactique ou d'ester d'acide lactique à déshydrater comprend de l'acide lactique ou un ou plusieurs ester(s) d'acide lactique provenant des étapes de recyclage d'un procédé de préparation de PLA, seul ou en mélange avec de l'acide lactique obtenu par fermentation directe.

2. Procédé selon la revendication 1, **caractérisé en ce que** la charge d'acide lactique ou d'ester d'acide lactique comprend de 5 à 100% en poids d'acide lactique ou d'ester d'acide lactique par rapport au poids total de la charge d'acide lactique ou d'ester d'acide lactique, provenant des étapes de recyclage d'un procédé de production de PLA.

3. Procédé selon la revendication 2, **caractérisé en ce que** la charge d'acide lactique ou d'ester d'acide lactique comprend de 50 à 100% en poids d'acide lactique par rapport au poids total de la charge d'acide lactique ou d'ester d'acide lactique provenant des étapes de recyclage d'un procédé de préparation de PLA.

4. Procédé selon les revendications 1 à 3, **caractérisé en ce que** le catalyseur de déshydratation est choisi dans le groupe comprenant les dérivés phosphatés de l'aluminium et les catalyseurs de type zéolithes ou tout autre catalyseur connu pour la déshydratation de l'acide lactique en acide acrylique.

5. Procédé selon la revendication 4, **caractérisé en ce que** le catalyseur de déshydratation est choisi parmi des zéolithes de type NaY échangées ou pas avec NaOH et traitée avec du baryum.

6. Procédé selon la revendication 4, **caractérisé en ce que** le catalyseur de déshydratation est choisi parmi des zéolithes de type ZSM-5 échangées avec de l'acide phosphorique.

7. Procédé selon la revendication 1, **caractérisé en ce que** la charge d'acide lactique à déshydrater est convertie en ester d'acide lactique.

8. Procédé selon la revendication 7, **caractérisé en ce que** la charge d'acide lactique à déshydrater est convertie en lactate d'éthyle.
